# EUROPEAN PATENT SPECIFICATION

(11) **EP 1 269 926 B1**
(45) Date of publication and mention of the grant of the patent: **21.05.2008**
(21) Application number: 02254541.2
(22) Date of filing: 27.06.2002
(51) Int. Cl.: A61B 17/34

(54) **Trocar having an improved seal design**
Trokar mit verbesserter Dichtungsausführung
Trocart avec une conception ameliorée de joint

(30) Priority: 28.06.2001 US 893390
(43) Date of publication of application: 02.01.2003
(73) Proprietor: Ethicon Endo-Surgery, Inc., Cincinatti, OH 45242 (US)
(72) Inventor: Tsonton, Mark, Loveland, Ohio 45140 (US); Voegele, Aaron C., Loveland, Ohio 45140 (US); Harper, Kevin A., Mason, Ohio 45040 (US)
(74) Representative: Mercer, Christopher Paul

(56) References cited:
- EP-A- 0 638 290
- WO-A-00/57939
- US-A- 5 342 315
- US-A- 5 662 615

## Description

### Field of the Invention

The present invention relates, in general, to trocars to assist in performing minimally invasive surgery and, more particularly, to improved seals for such trocars.

### Background of the Invention

The use of endoscopic procedures in surgery has become widely accepted. The term endoscopic as used herein is defined to include all types of minimally invasive surgical procedures including laparoscopic and arthroscopic procedures. Accordingly, numerous endoscopic instruments have been developed which allow the surgeon to perform complex surgical procedures with minimal incisions into the skin and tissue surrounding a particular body cavity or anatomical region. In order to introduce the endoscopic instrumentation into the body cavity, it is often necessary to puncture and cannulate the body cavity by using a trocar. Trocars are widely known in the art and typically consist of an obturator and a trocar cannula. An example of a trocar can be found in U.S. Patent 6,017,356 issued to Frederick et al. on January 25, 2000.

It is common for a sealing arrangement or sealing device to be used in association with the cannula to prevent the escape of fluid or gas during endoscopic procedures. During an endoscopic surgical procedure, the internal gas pressure must be maintained in order to successfully complete the procedure. In order to maintain the internal gas pressure while instruments are passed into and out of the trocars positioned in a body cavity, sealing devices are required for both the instruments and for the trocar assemblies. That is most trocars have two sealing devices. One which seals the trocar when there is not an instrument passing therethrough, and one which seals the trocar as instruments are passed therethrough. Furthermore, it is desirable that the sealing device maintain gas pressure in the abdominal cavity, despite numerous insertions and withdrawals of surgical instruments through the trocar cannula.

Most commercially available trocars have an outer seal and an inner seal. The outer seal is typically a gasket located at the proximal most end of the trocar cannula. This gasket tightly fits itself around the elongated shafts of any medical devices passing therethrough. Therefore, the outer seal prevents fluids from escaping the body cavity through the trocar cannula while surgical instruments are being used with the cannula. The inner seal is typically what is referred to as a flapper door. It is made from a rigid, typically plastic, door which is spring biased against an inner gasket. The inner seal prevents fluids from escaping the body cavity through the trocar cannula while the trocar cannula is not in use, i.e. with no surgical instruments or obturators passing therethrough. The inner seal is located with the trocar cannula handle, distal to the outer seal.

While for the most part the above design works well, there has been a tendency for the outer seal, or gasket, to tear during surgery. If a physician were to align a surgical instrument directly over the outer gaskets opening, with the longitudinal axis of the instrument parallel with the longitudinal axis of the trocar cannula, tearing would not be an issue. However, often times the physician enters the trocar cannula with the surgical instrument at an angle with respect to the cannula. This often causes the tip of the surgical instrument to be forced upon the wall of the gasket causing the gasket to tear. A torn gasket can lead to leaks during the surgical procedure, and may require the physician to insert a new cannula.

Others have tried to solve this problem. One attempted solution was a floating gasket, wherein the outer gasket floated on a bellows type structure. Examples of this type of seal are given in U.S. Patents 5,209,737 issued to Ritchart et al. on May 11, 1993; 5,385,553 issued to Hart et al on January 31, 1995; and 5,308,336 issued to Hart et al. on May 3 1994. However, this type of floating seal does not always significantly reduce the incidents of tearing. In addition, these types of seals can often be expensive.

Therefore, there has been a desire for an improved trocar seal design which significantly reduces the incidents of tearing during surgical procedures. There has also been a desire to provide such a design in a cost effective manner. A trocar seal design having the features set out in the preamble of claim 1 appended hereto is disclosed in US 5342315, and also in WO 00/57939.

### Summary of the Invention

The present invention as defined in claim 1 provides a trocar for performing a surgical procedure on a patient. The trocar includes a hollow cannula having a distal end and a proximal end, and a housing having a distal end attached to the proximal end of the cannula and a proximal end having a wall attached thereto. The wall has an aperture therethrough for inserting surgical instruments into the trocar. The trocar includes a hollow tubular guide member having a proximal end attached to the wall about the aperture and a distal end extending therefrom The trocar has a first seal comprising an elastomeric conical member having a proximal end adjacent to the distal end of the guide member, and a distal end extending therefrom, the distal end of the conical member having an opening therethrough. The trocar also has a second seal having an open position and a closed position, wherein when the second seal is in the open position there is fluid communication between the aperture and the cannula, and wherein when the second seal is in the closed position, fluid communication between the aperture and the cannula is substantially prevented.

### Brief Description of the Drawings

The novel features of the invention are set forth with particularity in the appended claims. The invention itself, however, both as to organization and methods of operation, together with further objects and advantages thereof, may best be understood by reference to the following description, taken in conjunction with the accompanying drawings in which:
FIG. 1 is a perspective view of a trocar assembly including the improved seal design of the present invention.
FIG. 2 is an exploded perspective view of the trocar assembly showing the obturator and trocar separated.
FIG. 3 is an interior view of the sleeve assembly of the present invention.
FIG. 4 is a cross-sectional view taken along line 4-4 of FIG. 2 of the improved seal design of the present invention showing the initial insertion of an instrument at an angle with respect to the trocar.
FIG. 5 is a cross-sectional view taken along line 4-4 of FIG. 2 of the improved seal design of the present invention showing the complete insertion of an instrument into the trocar.

### Detailed Description of the Invention

Reference numerals are used in this description to designate the various components and elements of the instrument of this invention. Identical reference numerals designated in the various drawings refer to the identical element or component of the surgical penetration instrument. As used in this description, "proximal" or "proximally" refers to that portion of the instrument, component, or element which extends toward the user. Conversely, "distal" or "distally" refers to that portion of the instrument, component, or element which extends away from the user.

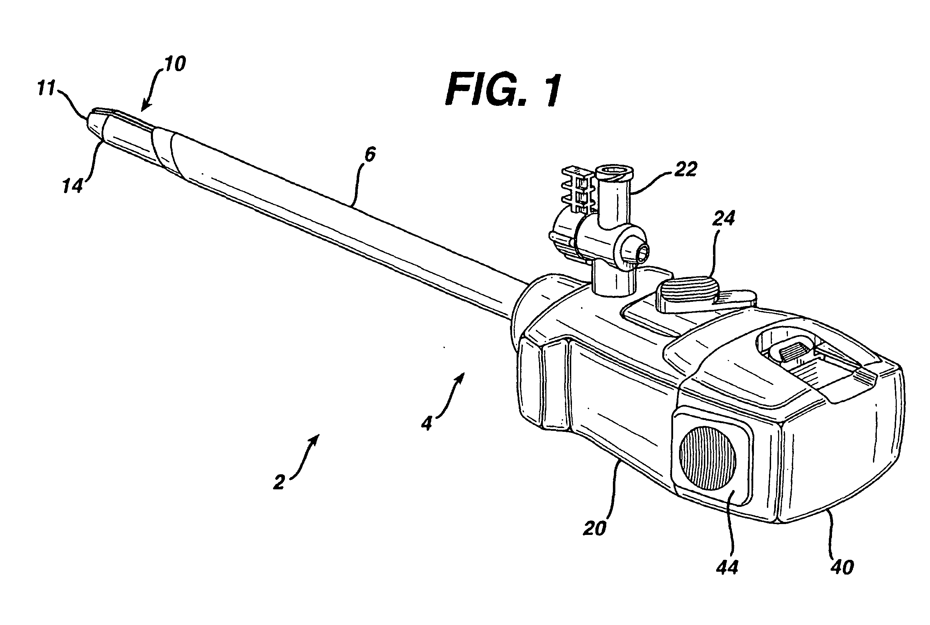

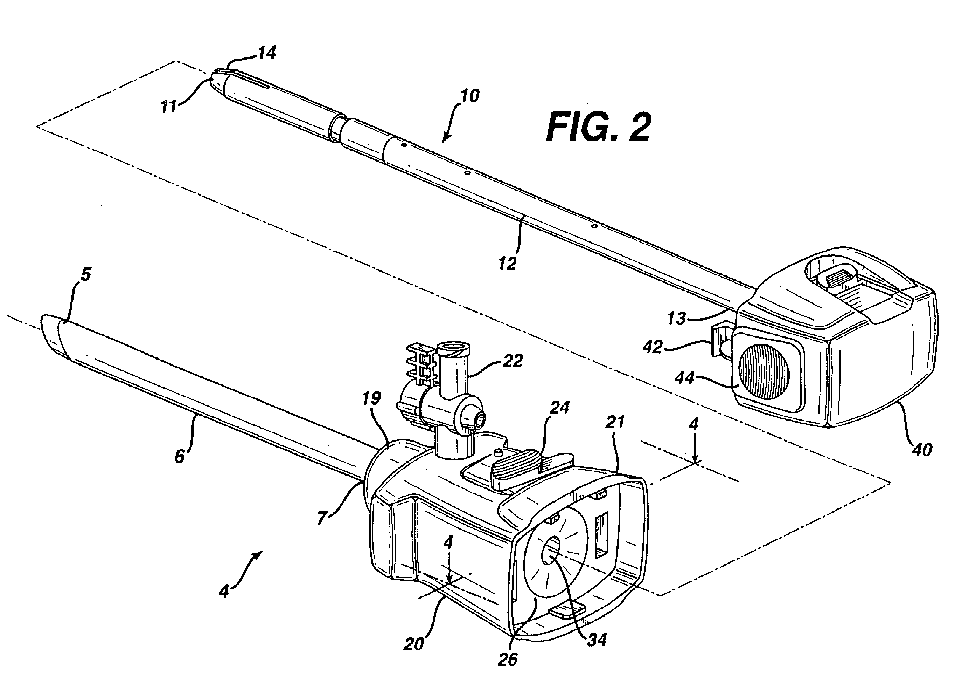

Referring to FIGS. 1 and 2, there is shown trocar 2 made in accordance with the present invention. Trocar 2 includes a sleeve assembly 4 and obturator 10. As seen from the figures, obturator 10 includes an elongated shaft 12 having a distal end 11 and a proximal end 13. Distal end 11 has a penetrating tip 14 disposed thereon. Penetrating tip 14 is a safety shielded penetrating tip and is well known in the art and is described in the hereinbefore incorporated references. Proximal end 13 of shaft 12 has a hub or handle 40 attached thereto. Hub 40 contains latch 42 which secures obturator 10 to sleeve assembly 4. Buttons 44 and 46 (not shown) are connected to latch 42.

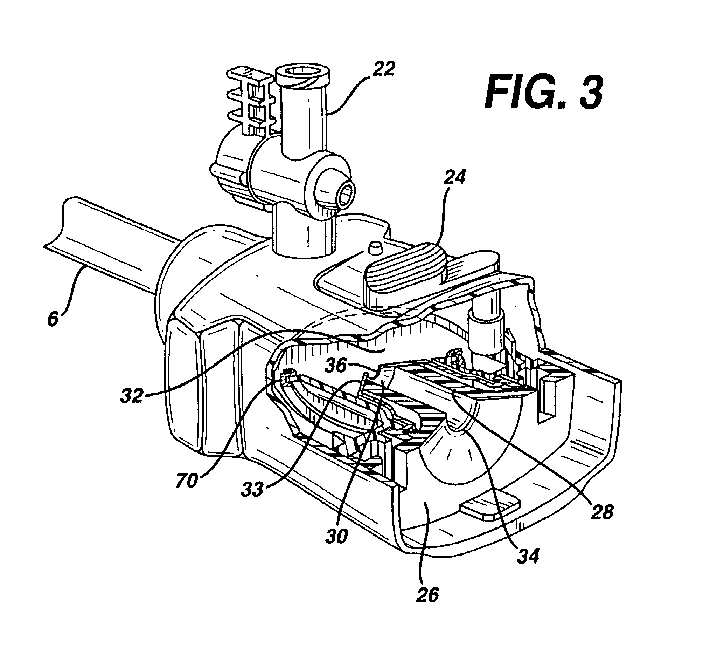

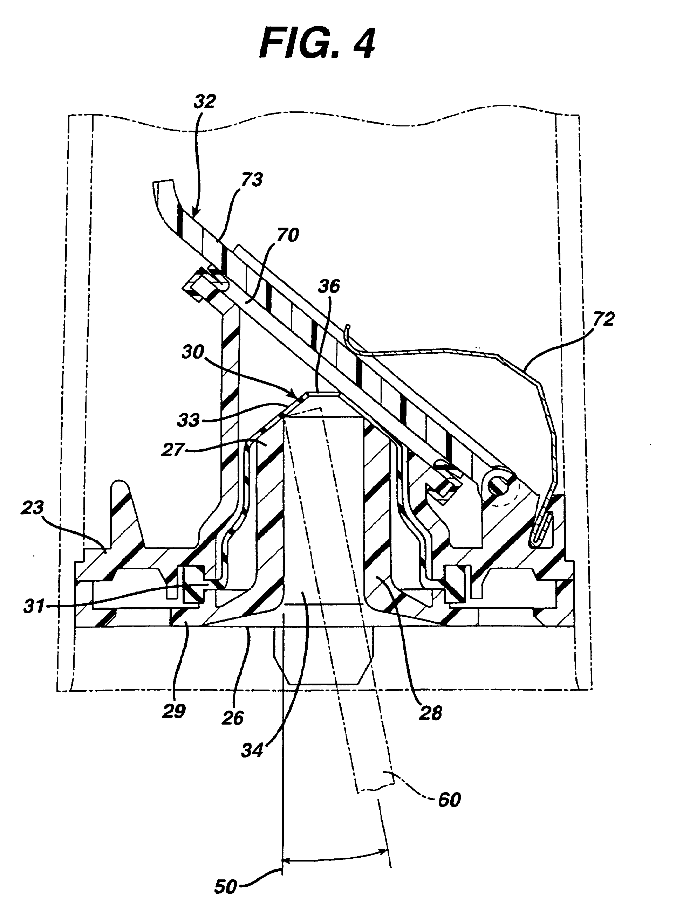

Sleeve assembly 4 of trocar 2 includes a hollow cannula 6 having a distal end 5 and a proximal end 7. The trocar further includes a generally hollow housing 20 having a distal end 19 attached to proximal end 7 of cannula 6, whereby cannula 6 extends distally from housing 20. Housing 20 further includes a proximal end 21 having a wall 26 attached thereto. Wall 26 has an aperture 34 extending therethrough. Referring now to Figures 3 and 4, it can be understood that the trocar 2 further includes a hollow tubular guide member 28. Member 28 has a proximal end 29 is attached to wall 26 about aperture 34. Member 28 further includes a distal end 27 extending away from wall 26.

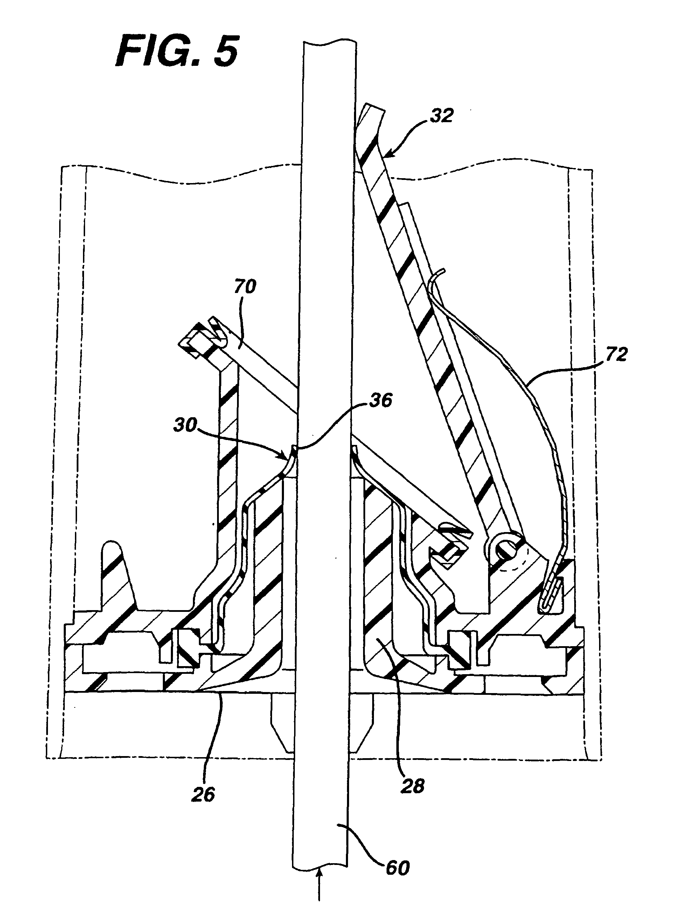

Trocar 2 includes a first seal 30 which is adjacent to the distal end 27 of member 28. First seal 30 is an elastomeric conical member and can be made from silicone, polyisoprene, or any other material known to those skilled in the art. First seal 30 has a proximal end 31 which is sandwiched between member 28 and frame 23 of housing 20 for securement. First seal 30 includes opening 36 at its distal end 33. Housing 20 further includes a second seal 32 immediately adjacent to distal end 33 of seal 30 but not abutting. Second seal 32 includes a gasket 70 fixedly secured to frame 23, and flapper door 73 pivotably secured to frame 23. Flapper door 73 is biased against gasket 70 by a spring 72 attached to frame 23. Spring 72 can be made from any elastic material, such as stainless steel, which recovers somewhat upon being deformed. Second seal 32 has an open position (shown in FIG. 5) and closed position (shown in FIG. 4). When second seal 32 is in an open position, there is fluid communication between aperture 34 and cannula 6. When second seal 32 is in a closed position, fluid communication between aperture 34 and cannula 6 is substantially prevented. Lastly, the longitudinal spacing between the flapper door 73 and distal end 33 of seal 30 is between about 6.35 mm to about 1.27 mm (about 0.25 inch to about 0.05 inch).

When using trocar 2 in a surgical procedure, obturator 10 is inserted through aperture 34 into housing 20 and cannula 6. When obturator 10 is fully inserted into sleeve assembly 4, hub 40 is secured to housing 20 by latch 42. Penetrating tip 14 of obturator 10 extends distally from cannula 6. In an actual surgical procedure utilizing the device of the present invention, a surgeon, using a scalpel, makes a small incision where trocar 2 is to be positioned during the surgical procedure. The distal end of trocar 2 is then inserted into the tissue exposed by the small incision. After insertion into the tissue, trocar 2 is advanced into the interior of a body, such as the abdominal cavity.

After penetration into the abdominal cavity is complete, obturator 10 is removed from sleeve assembly 4 by pressing buttons 44 and 46 (not shown) which releases latch 42 from housing 20. When obturator 10 is removed second seal 32 connected to valve lever 24 closes. After closing, second seal 32 is retained upon gasket 70 by flapper spring 72 to prevent any fluid or gas from escaping sleeve assembly 4. If desired, a pressurizing gas such as carbon dioxide can be selectively pumped through cannula 6 via stopcock 22 to insufflate the surgical site.

As shown in FIG. 4, an instrument 60, such as a linear stapler, grasper, clip applier, scope etc., can then be inserted through aperture 34 into sleeve assembly 4 to perform a procedure at the surgical site. Often, instrument 60 is blindly inserted into housing 20 by a surgeon during the surgical procedure. This causes instrument 60, as illustrated in FIG. 4, to be inserted into housing 20 at an angle with respect to longitudinal axis 50. To reduce the angle of insertion, hollow tubular guide member 28 aligns instrument 60 substantially parallel with longitudinal axis 50 of sleeve assembly 4 before sealing occurs. For example, using a 3 mm trocar and a 2.2 mm instrument, guide member 28 of the present invention would reduce the angle of insertion into first seal 30 as great as 40.2 degrees. This decreases the risk of tearing first seal 30 which tightly fits itself around instrument 60 to prevent any fluid or gas from escaping sleeve assembly 4.

The greater the length of the guide member 20, the more the instrument will become aligned before reaching the seal 30. However, surgeons prefer to have smaller housings, as they might interfere with their work. Therefore, the length of member 20 is somewhat limited. However, the conical design of first seal 30 helps to increase this longitudinal distance by placing the aperture 36 even deeper within the housing. As instrument 60 is advanced through first seal 30, its conical design helps to further reduce tearing by orientating instrument 60 to opening 36 rather than deflecting during insertion. Instrument 60 can then be inserted further into housing 20 causing second seal 32 connected to valve lever 24 to separate from gasket 70. After instrument 60 is completely advanced through housing 20, as shown in FIG. 5, flapper spring 72 retains second seal 32 against instrument 60 in an open position. Instrument 60 can then be completely inserted through cannula 6 to perform the desired procedure at the surgical site.

An additional benefit of sleeve assembly 4 of the present invention is a decrease in the drag on instrument 60 which thus causes an increase in the stability of trocar 2. One way to decrease the drag on instrument 60, is to increase the diameter of opening 36. This increase in the diameter of opening 36 can be achieved because the first seal 30 is positioned closer to center pivot point of instrument 60. Because the amount instrument 60 can be placed off axis is reduced, opening 36 can be larger. Having first seal 30 distal to guide member 28 increases the distance between aperture 34 and first seal 30. By increasing this distance, there is less leak from holding instrument 60 off center to opening 36 enabling an increase in its diameter.

While preferred embodiments of the present invention have been shown and described herein, it will be obvious to those skilled in the art that such embodiments are provided by way of example only. Numerous variations, changes, and substitutions will now occur to those skilled in the art without departing from the invention. Accordingly, it is intended that the invention be limited only by the scope of the appended claims.

## Claims

1. A trocar (2) for performing a procedure on a patient, said trocar (2) comprising:
a. a hollow cannula (6) having a distal end (5) and a proximal end (7);
b. a housing (20) having a distal end (19) attached to said proximal end (7) of said cannula (6) and a proximal end (21) having a wall (26) attached thereto, said wall (26) having an aperture (34) therethrough;
c. a hollow tubular guide member (28) having a proximal end (29) attached to said wall (26) about said aperture (34) and a distal end (27) extending therefrom;
d. a first seal (30) comprising an elastomeric conical member having a proximal end (31) adjacent to said distal end (27) of said guide member (28), and a distal end (33) extending therefrom, said distal end (33) of said conical member having an opening (36) therethrough; and
e. a second seal (32) having an open position and a closed position, wherein when said second seal (32) is in said open position there is fluid communication between said aperture (34) and said cannula (6), and wherein when said second seal (32) is in said closed position, fluid communication between said aperture (34) and said cannula (6) is substantially prevented;
the trocar being **characterised by**:
the second seal comprising a flapper door (73) pivotably secured to a frame (23) and spring biased against a gasket (70) that is also secured to the frame (23);
the second seal (32) being immediately adjacent to said distal end of said first seal, but not abutting;
the longitudinal distance between the distal end of said first seal and the flapper door (73) being between 6.35mm and 1.27mm (0.25" and 0.05"); and
the first seal (30) extending distally with respect to the hinge of the flapper door (73).

2. The trocar (2) of claim 1 wherein said aperture (34) has a diameter which is greater than a diameter of said opening (3b).

3. The trocar (2) of any one of claims 1 to 2, in combination with an instrument to be inserted therein, wherein, in use, said guide member (28) aligns the longitudinal axis of the instrument to be inserted therein substantially parallel with the longitudinal axis of said housing (20).

4. The trocar (2) of any one of claims 1 to 3, wherein said first seal (30) is comprised of silicone.

5. The trocar (2) of any one of claims 1 to 4, wherein said first seal (30) is comprised of polyisoprene.

## Patentansprüche

1. Trokar (2) zum Behandeln eines Patienten, umfassend:
a) eine hohle Kanüle (6) mit einem distalen Ende (5) und einem proximalen Ende (7);
b) ein Gehäuse (20) mit einem distalen Ende (19), das an dem proximalen Ende (7) der Kanüle (6) angebracht ist, und ein proximales Ende (21), das eine daran angebrachte Wand (26) aufweist, wobei die Wand (26) eine Öffnung (34) dadurch aufweist;
c) ein hohles, rohrförmiges Führungsbauteil (28) mit einem proximalen Ende (29), das an der Wand (26) um die Öffnung (34) herum angebracht ist, und einem distalen Ende (27), das sich davon erstreckt;
d) eine erste Dichtung (30), die ein konisches Elastomerteil mit einem proximalen Ende (31) benachbart dem distalen Ende (27) des Führungsbauteils (28) und einem distalen Ende (33) umfaßt, das sich davon erstreckt, wobei das distale Ende (33) des konischen Elastomerteils eine Öffnung (36) dadurch aufweist; und
e) eine zweite Dichtung (32) mit einer offenen Position und einer geschlossenen Position, wobei, wenn sich die zweite Dichtung (32) in der offenen Position befindet, eine Fluidverbindung zwischen der Öffnung (34) und der Kanüle (6) besteht, wobei, wenn die zweite Dichtung (32) in der geschlossenen Position ist, eine Fluidverbindung zwischen der Öffnung (34) und der Kanüle (6) im wesentlichen verhindert ist;
**dadurch gekennzeichnet, daß:**
die zweite Dichtung eine Klapptür (73) umfaßt, die schwenkbar an einem Rahmen (23) angebracht ist und gegen eine Dichtung (70) federvorgespannt ist, die ebenfalls an dem Rahmen (23) angebracht ist;
die zweite Dichtung (32) unmittelbar benachbart dem distalen Ende der ersten Dichtung liegt, aber gegen diese nicht stößt;
der Längsabstand zwischen dem distalen Ende der ersten Dichtung und der Klapptür (73) zwischen 6,35 mm und 1,27 mm (0,25 Inch und 0,05 Inch) liegt; und
sich die erste Dichtung (30) distal bezüglich dem Gelenk der Klapptür (73) erstreckt.

2. Trokar nach Anspruch 1, bei dem die Öffnung (34) einen Durchmesser aufweist, der größer als ein Durchmesser der Öffnung (36) ist.

3. Trokar nach Anspruch 1 oder 2, mit einem Gerät zum darin Einführen, wobei im Gebrauch das Führungsbauteil (28) der Längsachse des darin einzusetzenden Gerätes im wesentlichen parallel zur Längsachse des Gehäuses ausrichtet.

4. Trokar nach einem der Ansprüche 1 bis 3, bei dem die erste Dichtung (30) aus Silikon besteht.

5. Trokar nach einem der Ansprüche 1 bis 4, bei dem die erste Dichtung (30) aus Polyisopren besteht.

## Revendications

1. Trocart (2) destiné à exécuter une procédure sur un patient, ledit trocart (2) comprenant :
a. une canule creuse (6) présentant une extrémité distale (5) et une extrémité proximale (7) ;
b. un logement (20) présentant une extrémité distale (19) fixée à ladite extrémité proximale (7) de ladite canule (6) et une extrémité proximale (21) à laquelle est fixée une paroi (26), ladite paroi (26) présentant une ouverture (34) traversante ;
c. un élément de guidage tubulaire creux (28) présentant une extrémité proximale (29) fixée à ladite paroi (26) autour de ladite ouverture (34) et une extrémité distale (27) s'étendant à partir de celle-ci ;
d. un premier joint (30) comprenant un élément conique élastomère présentant une extrémité proximale (31) adjacente à ladite extrémité distale (27) dudit élément de guidage (28), et une extrémité distale (33) s'étendant à partir de celui-ci, ladite extrémité distale (33) dudit élément conique présentant un orifice (36) traversant ; et
e. un deuxième joint (32) présentant une position ouverte et une position fermée, dans lequel lorsque ledit deuxième joint (32) est dans ladite position ouverte, il existe une communication fluide entre ladite ouverture (34) et ladite canule (6) et dans lequel lorsque ledit deuxième joint (32) est dans ladite position fermée, une communication fluide entre ladite ouverture (34) et ladite canule (6) est sensiblement empêchée ;
le trocart étant **caractérisé par le fait que** :
le deuxième joint comprend une fermeture à clapet (73) fixée par pivotement à une armature (23) et appliquée par ressort contre une garniture (70) qui est aussi fixée à l'armature (23) ;
le deuxième joint (32) est immédiatement adjacent à ladite extrémité distale dudit premier joint, mais pas en butée ;
la distance longitudinale entre l'extrémité distale dudit premier joint et la fermeture à clapet (73) est comprise entre 6,35 mm et 1,27 mm (0,25" et 0,05") ; et
le premier joint (30) s'étend de manière distale par rapport à l'articulation de la fermeture à clapet (73).

2. Trocart (2) selon la revendication 1, dans lequel ladite ouverture (34) présente un diamètre qui est supérieur au diamètre dudit orifice (36).

3. Trocart (2) selon l'une quelconque des revendications 1 à 2, en association avec un instrument à insérer à l'intérieur, dans lequel, en utilisation, l'élément de guidage (28) aligne l'axe longitudinal de l'instrument à insérer à l'intérieur de manière sensiblement parallèle à l'axe longitudinal dudit logement (20).

4. Trocart (2) selon l'une quelconque des revendications 1 à 3, dans lequel ledit premier joint (30) est composé de silicone.

5. Trocart (2) selon l'une quelconque des revendications 1 à 4, dans lequel ledit premier joint (30) est composé de polyisoprène.
